# EUROPEAN PATENT APPLICATION

(11) **EP 0 753 652 A1**
(43) Date of publication of application: **15.01.1997**
(21) Application number: 95201885.1
(22) Date of filing: 10.07.1995
(51) Int. Cl.: F02C 6/10, C07C 11/04

(54) **Synthesis of ethene**

(71) Applicant: N.V. Kema, NL-6812 AR Arnhem (NL)
(72) Inventor: Janssen, Frans Johan Joseph Gerard, NL-6814 KV Arnhem (NL); Verkooijen, Adrianus Hubertus Maria, NL-6865 TJ Doorwerth (NL); Ploumen, Pieter Joseph, NL-6605 HP Wychen (NL)
(74) Representative: Van Someren, Petronella F. H. M.

(57) **Abstract**

Proces for the synthesis of ethene (≡ethylene, C₂H₄) from methane. Exothermic catalytic combustion in combustion chamber of gasturbine with exhaust products driving turbine to compress methane and combustion air and produce electricity. After driving turbine exhaust products separated by membrane separator into ethene stream and byproducts stream (including CO,CO₂,H₂O).

## Description

This invention relates to a process for producing energy and to a system therefor.

Electricity, for instance, is presently generated on a large scale, by combusting volatile fuels in the presence of hot air in the combustion chamber of a gas turbine, whereafter the resulting hot gases are used to drive the turbine whose rotational energy is subsequently converted via a generator into electricity.

An object of the present invention is to provide an alternative process for providing energy.

Many chemical reactions carried out in the petrochemical industry are exothermic, i.e. heat is released on the formation of products.

In many such petrochemical reactions, this reaction heat is either cooled down and released into the environment or converted into useful energy via heat exchanges.

According to a first aspect of the present invention, there is provided a process for producing energy, comprising the steps of:
a) carrying out at least one exothermic reaction;
b) harnessing at least a substantial amount of the heat released from said exothermic reactions; and
c) displacing a substantial amount of the heat harnessed in step b) in order to generate a kinetic energy stream.

Hence, energy released from exothermic reactions is harnessed as a kinetic energy stream, which can thereafter be utilized in any desirable way, for instance in producing steam, buy is preferably utilized in the generation of electricity by directing it through a turbine.

Preferably, the kinetic energy stream transports at least one of the products, of the exothermic reactions, with it through the turbine.

In this way the steam is expanded whereby its kinetic energy is provided.

Preferably at least one of the products, of the exothermic reactions, is recovered from the kinetic energy stream after the passing thereof through the turbine.

Accordingly at least one chemical product is obtained.

Preferably the heat released in the exothermic reactions, originates from carrying out at least one of the following reactions, as these are energetically favorable.

| No. | Reaction |
|---|---|
| 1 | CH₄ + 1/4O₂ = 1/2C₂H₆ + 1/2H₂O |
| 2 | CH₄ + 1/2O₂ = 1/2C₂H₄ + H₂O |
| 3 | CH₄ + 2O₂ = CO₂ + 2H₂O |
| 4 | 2CH₄ + O₂ = 2CO + 4H₂ |
| 5 | 2SO₂ + O₂ = 2SO₃ |
| 6 | CO + 2H₂ = CH₃OH |
| 7 | 2C₂H₄ + O₂ = 2CO₂H₄O |
| 8 | C₂H₂ + h₂ = C₂H₄ |
| 9 | 4NH₃ + 5O₂ = 4NO + 6H₂O |

Since the reactions are exothermic, they show selfignition and hence no spark is needed to ignite the reaction mixture.

Methane is preferably used as this offers the following advantages;
- it can be converted into hydrocarbons, which are more economically transportable than methane;
- the direct production of chemicals from methane can take place more selectively, via the present invention, than with existing processes such as steam reforming and Fisher-Tropsch;
- and methane is more economically favorable converted into ethene, using the process according to the present invention, than with current processes.

Most preferably is reaction 2, as ethene, a desirable product, is yielded.

According to further aspects of the present invention there is provided a process for obtaining chemical products; a system for providing energy; a system for generating electricity; and the use of heat resulting from exothermic reactions as kinetic energy, which may be used for any desired application such as driving a turbine in order to generate electricity.

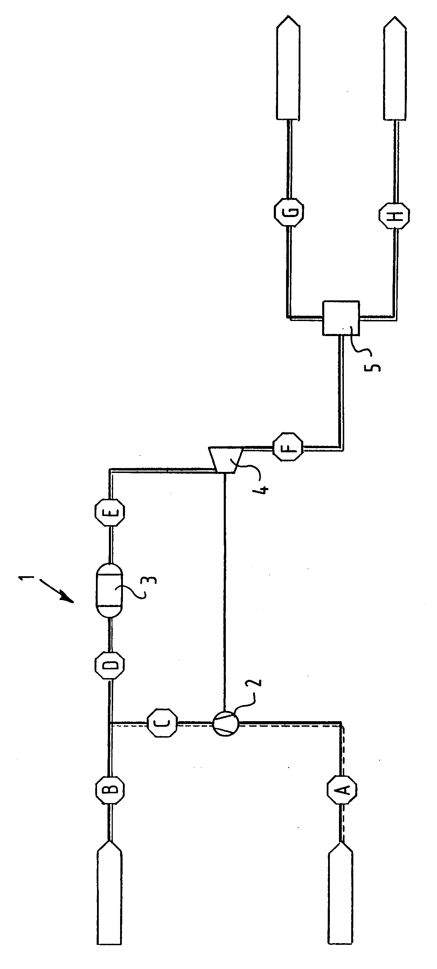

The invention will now be elucidated by the following non limiting description, experimental results and figure 1 which shows, schematically, a preferred embodiment of the system for generating electricity according to the present invention.

The system 1 (see figure) comprises a compressor 2 an adiabatic chemical reactor 3, a turbine 4 and a separation unit 5.

In use, air, stream A, is pressurized form 1 bar to 10 bar in the compressor 2, to yield stream C which is thereafter mixed with methane, stream B, at this pressure.

The ensuing mixture, stream D, is passed to the adiabatic chemical reactor 3, where exothermic chemical reactions take place, preferably over a catalyst, preferably a lythium/magnesium oxide catalyst, this being the most suitable, whereby the temperature of the resulting gas mixtures increases.

Utilizing an adiabatic chemical reactor means that substantially no heat enters or leaves the reactor via conduction through the reactor walls, as the reactor, being adiabatic, is unable to transmit energy through its walls. Hence the rise in internal energy of the reacting gas mixture appears as a rise in the temperature of the compressed gas, and is stored as thermal motion.

The resulting gas mixture, stream E, drives the turbine 4 by expanding through the latter as the pressure is lowered from 10 bar down to 1 bar.

After passing through the turbine 4, the gas mixture, stream F, is then separated by a separation unit 5, which preferably takes the form of a membrane into ethene, stream G, and a mixture of CO, CO₂, H₂O and CH₄ stream H.

This latter mixture, stream H, can be recycled back into the system to take advantage of any combustion value therein, which can be utilized by underfiring in the system for example.

Tests were carried out by the inventors on the system as shown in figure 1 and the following results were obtained.

Table 1, summarizes the results measured.

**Table 1**

| | Value | Unit |
|---|---|---|
| Heat input | 500551 | [kW] |
| Heat residual gas, stream G, fig.1 | 335840 | [kW] |
| Energy content ethene | 51143 | [kW] |
| Electricity production | 18771 | [kW] |
| Heat production in chemical reactor | 92824 | [kW] |
| Electric efficiency | 3,75 | [%] |
| Heat efficiency | 18,52 | [%] |
| Yield ethene efficiency | 10,22 | [%] |
| Yield residual gas efficiency | 67,09 | % |

Table 2, shows the results of flow rate, pressure, temperature and enthalpy measurements for the streams A to H.

**Table 2**

| stream | flow (kg/s) | press. (bar) | temp. (°C) | enthalpy (kJ/kg) |
|---|---|---|---|---|
| A | 64.80 | 1.0130 | 15.0 | -57.8 |
| B | 10.00 | 10.0000 | 45.0 | -4629.3 |
| C | 64.80 | 10.0000 | 320.2 | 256.5 |
| D | 74.80 | 10.0000 | 242.8 | -396.6 |
| E | 74.80 | 9.9000 | 850.0 | -1637.6 |
| F | 74.80 | 1.2000 | 496.9 | -2177.2 |
| G | 1.06 | 1.1900 | 496.9 | 2960.6 |
| H | 73.74 | 1.1900 | 496.9 | -2251.1 |

Table 3 shows the gas composition measured for streams A to H.

**Table 3**

| Stream | A, C | B | D | E, F | H | G |
|---|---|---|---|---|---|---|
| Ar | 0.00926 | | 0.00724 | 0.00691 | 0.00700 | |
| O₂ | 0.20483 | | 0.16308 | 0.00000 | 0.00000 | |
| N₂ | 0.77683 | | 0.60781 | 0.58024 | 0.58764 | |
| H₂O | 0.00519 | | 0.00406 | 0.21762 | 0.22040 | |
| CO | | | | 0.09070 | 0.09185 | |
| CO₂ | 0.00030 | | 0.00024 | 0.00368 | 0.00373 | |
| CH₄ | | 1.00000 | 0.21758 | 0.08812 | 0.08925 | |
| C₂H₄ | | | | 0.01272 | 0.00013 | 1.00000 |
| Avmw [kg/mol] | 28.9098 | 16.0430 | 26.1102 | 24.9261 | 24.8863 | 28.0540 |

From these results, 10 kg/s of methane can be transformed into roughly 1 kg/s of ethene via the present invention.

Furthermore about 18.8 MWe and 92.8 MWth become available in heat at a temperature of round about 850°C.

The present invention is not limited by the above described system and results, but its scope is rather determined by the following claims.

## Claims

1. Process for producing energy comprising the steps of:
a) carrying out at least one exothermic reaction;
b) harnessing at least a substantial amount of the heat released from said exothermic reactions; and
c) displacing a substantial amount of the heat harnessed in step b) in order to generate a kinetic energy stream.

2. Process according to claim 1, wherein the reaction mixture shows selfignition.

3. Process according to claims 1 or 2 wherein the kinetic energy stream is directed through a turbine in order to generate electricity.

4. Process according to any of the claims 1 to 3 wherein at least one of the reaction products, yielded by the exothermic reactions, is transported by the kinetic energy stream.

5. Process according to claim 4 wherein said reaction product is recovered after the kinetic energy stream has substantially passed through the turbine.

6. Process according to any preceding claim, wherein heat is released by carrying out at least one of the following exothermic reactions
| No. | Reaction |
|---|---|
| 1 | CH₄ + 1/4O₂ = 1/2C₂H₆ + 1/2H₂O |
| 2 | CH₄ + 1/2O₂ = 1/2C₂H₄ + H₂O |
| 3 | CH₄ + 2O₂ = CO₂ + 2H₂O |
| 4 | 2CH₄ + O₂ = 2CO + 4H₂ |
| 5 | 2SO₂ + O₂ = 2SO₃ |
| 6 | CO + 2H₂ = CH₃OH |
| 7 | 2C₂H₄ + O₂ = 2CO₂H₄O |
| 8 | C₂H₂ + h₂ = C₂H₄ |
| 9 | 4NH₃ + 5O₂ = 4NO + 6H₂O |
and preferably reaction no. 2.

7. System for applying the method as claimed in claim 1, comprising:
- at least one compressor for compressing a substantially air stream
- at least one reactor wherein exothermic reactions can take place, said reactor not being provided with a sparking device for igniting reaction mixtures.

8. System according to claim 7, wherein the reactor is an adiabatic chemical reactor.

9. System acording to claim 7 or 8 for, in addition to applying the method as claimed in claim 1, applying the method as claimed in any of the claims 3 to 6, further comprising:
- at least one turbine
- at least one separation unit for separating gas mixtures into desired streams.

10. System according to claim 9 wherein the separation unit comprises at least one membrane.

11. Use of heat resulting from exothermic reactions as a kinetic energy stream, which can be used to drive a turbine thereby generating electricity.

12. Use of the system according to claim 7 for providing a kinetic energy stream.

13. Use of the system according to claims 9 or 10 for generating electricity.

14. Method for yielding at least one chemical product, in particular ethene comprising the steps of:
- reacting a pressurized air and methane stream in an adiabatic chemical reactor,
- expanding the products of this reaction through a turbine,
- separating the products into an ethene stream and a residual stream.

15. Use of the system according to claims 9 or 10 for applying the method according to claim 14.
